# EUROPÄISCHE PATENTSCHRIFT

(11) **EP 4 138 627 B1**
(45) Veröffentlichungstag und Bekanntmachung des Hinweises auf die Patenterteilung: **22.10.2025**
(21) Anmeldenummer: 21720739.8
(22) Anmeldetag: 21.04.2021
(51) Int. Cl.: A61B 1/00, A61B 1/267

(54) **MEDIZINISCHE VORRICHTUNG**
MEDICAL DEVICE
DISPOSITIF MÉDICAL

(30) Priorität: 21.04.2020 DE 102020110845
(43) Veröffentlichungstag der Anmeldung: 01.03.2023
(73) Patentinhaber: Freitag, Lutz, 58675 Hemer (DE)
(72) Erfinder: Freitag, Lutz, 58675 Hemer (DE)
(74) Vertreter: Griepenstroh, Jörg
(86) Internationale Anmeldenummer: PCT/EP2021/060368
(87) Internationale Veröffentlichungsnummer: WO 2021/214132

(56) Entgegenhaltungen:
- EP-A1- 3 494 913
- DE-A1- 4 444 049
- DE-U1- 202012 007 821
- US-A1- 2015 335 228

## Beschreibung

Die Erfindung betrifft eine medizinische Vorrichtung mit den Merkmalen des Patentanspruchs 1 .

Endoskope werden für minimalinvasive, operative Eingriffe an Menschen und Tieren, sowie in der Technik zur Sichtprüfung schwer zugänglicher Hohlräume eingesetzt. Je nach Verwendungszweck kommen Endoskoprohre mit unterschiedlichen Durchmessern zum Einsatz. In der Bronchoskopie kommen oftmals starre Bronchoskoprohre aus Edelstahl mit Wanddicken von 0,6 mm zum Einsatz. Durchmesser im Bereich von 10 mm mit Längen von ca. 400 mm sind üblich. Zum Platzieren der häufig verwendeten Silikonstents sind Rohrdurchmesser von 10 - 16 mm erforderlich entsprechend der Weite eines normalen Kehlkopfes. Der Umgang mit dickeren Geräten zur Stenteinlage erfordert Übung und Erfahrung.

Ein weiteres Problem besteht darin, dass bei der Endoskopie in der Regel nicht ohne größeren Aufwand von einem kleineren Durchmesser auf einen größeren Durchmesser (oder umgekehrt) gewechselt werden kann. In der Regel ist es hierzu erforderlich, das Endoskoprohr einschließlich des daran befestigten Endoskopkopfes komplett zu tauschen und alle daran angeordneten Anschlüsse neu zu befestigen.

Zum Stand der Technik wird auf DE 44 44 049 A1 hingewiesen, die eine medizinische Vorrichtung mit einem Endoskopkopf offenbart, der dazu ausgebildet ist, mit Endoskoprohren lösbar verbunden zu werden. Der Endoskopkopf besitzt einen Längskanal zur Aufnahme eines Endoskoprohrs. Der Längskanal besitzt eine variable Öffnungsweite zur Aufnahme von Endoskoprohren unterschiedlicher Durchmesser. Zudem besitzt der Endoskopkopf eine Spannvorrichtung mit mehreren Spannkörpern, um ein in den Längskanal eingesetztes Endoskoprohr klemmend zu halten. Die Spannkörper sind über den Umfang des Längskanals verteilt angeordnet und können von radial außen gegen das Endoskoprohr gedrängt werden. Sie sind mit einem gemeinsamen Grundkörper verbunden.

Weitere Bauformen von Bronchoskopieadaptern sind der US 2015/0335228 A1 und der EP 3 494 913 A1 zu entnehmen.

Der Erfindung liegt die Aufgabe zu Grunde, eine medizinische Vorrichtung mit einem Endoskopkopf aufzuzeigen, der es ermöglicht, auf alternative Weise Endoskoprohre unterschiedlicher Durchmesser zu fixieren.

Die Aufgabe ist bei einer medizinischen Vorrichtung mit den Merkmalen des Patentanspruchs 1 gelöst.

Die Unteransprüche betreffen vorteilhafte Weiterbildungen der Erfindung.

Die medizinische Vorrichtung umfasst einen Endoskopkopf, der dazu ausgebildet ist, mit Endoskoprohren lösbar verbunden zu werden. Der Endoskopkopf besitzt hierfür einen Längskanal zur Aufnahme eines Endoskoprohrs.

Die Erfindung hat nicht zum Gegenstand, eine unlösbare Verbindung zwischen dem Endoskopkopf und dem Endoskoprohr aufzuzeigen, sondern betrifft maßgeblich die Gestaltung des Endoskopkopfes, der beispielsweise nach der Verbindung mit dem Endoskoprohr und nach Abschluss der Untersuchung oder Behandlung dafür vorgesehen ist, wieder von dem Endoskoprohr getrennt zu werden.

Das Besondere an dem Endoskopkopf ist, dass der Längskanal zumindest bereichsweise eine variable Öffnungsweite zur Aufnahme von Endoskoprohren unterschiedlicher Durchmesser besitzt. Anders als Endoskopköpfe, die beispielsweise mit unterschiedlich langen Endoskoprohren verbunden werden können, aber einen einheitlichen Durchmesser besitzen, besitzt der Endoskopkopf eine variable Öffnungsweite. Die Öffnungsweite ist an die Durchmesser der aufzunehmenden Endoskoprohre anpassbar.

Ferner besitzt der Endoskopkopf eine Spannvorrichtung, um ein in den Längskanal eingesetztes Endoskoprohr klemmend zu halten. Die Spannvorrichtung hat zwei Aufgaben: erstens soll sie das Endoskoprohr klemmend halten und zweitens soll sie die Öffnungsweite des Längskanals im Endoskopkopf an den jeweiligen Durchmesser anpassen. Die Spannvorrichtung dient darüber hinaus vorzugsweise dazu, das Endoskoprohr dicht und insbesondere gasdicht in dem Längskanal zu halten. Das kann unter Eingliederung von Dichtmitteln erfolgen, die einen Spalt zwischen dem Endoskoprohr und dem Endoskopkopf abdichten.

Die Spannvorrichtung kann im unbelasteten, d.h. im nicht gespannten Zustand, eine Offenstellung einnehmen, d.h. die maximale Öffnungsweite freigeben. Das Spannen der Spannvorrichtung zum Erzeugen der Klemmwirkung erfolgt über eine Krafteinwirkung von außen auf die Spannvorrichtung, so dass sich die Öffnungsweite verringert.

Die Spannvorrichtung besitzt mehrere verlagerbare Spannkörper. Es handelt sich vorzugsweise um wenigstens drei Spannkörper, die insbesondere gleichmäßig über den Umfang des Längskanals verteilt angeordnet sind. Die mehreren verlagerbaren Spannkörper ermöglichen eine Zentrierung des Endoskoprohrs in dem Längskanal.

Die Spannkörper sind von radial außen gegen das aufgenommene Endoskoprohr zwängbar oder drängbar. Die Klemmwirkung erfolgt durch radialen Druck, der von außen über die Spannkörper auf die Wand des Endoskoprohrs ausgeübt wird. Die insbesondere gleichmäßig über den Umfang verteilt angeordneten Spannkörper werden bevorzugt alle gleichmäßig verlagert, und besitzen insbesondere dieselbe Geometrie.

Die Spannkörper sind beweglich mit einem gemeinsamen Grundkörper verbunden.

Dadurch sind die Spannkörper unverlierbar mit dem Grundkörper gekoppelt. Ein Führungskäfig ist nicht erforderlich.

Der Grundkörper selbst kann Bestandteil des Endoskopkopfes sein oder mit diesem verbunden sein. Bevorzugt sind die Spannkörper, der Grundkörper und auch der gesamte Endoskopkopf materialeinheitlich einstückig hergestellt, bspw. aus einem geeigneten Kunststoff. Die Materialeigenschaften können in Abhängigkeit von der Funktion der einzelnen Bereiche variieren. Bspw. können die einzelnen Spannkörper im Übergangsbereich zu dem Grundkörper, an dem sie befestigt sind, aus einem nachgiebigeren Material bestehen und in denjenigen Bereichen, in denen die Kraft zwischen dem Endoskoprohr und dem Endoskopkopf übertragen werden muss, aus weniger nachgiebigem Material bestehen. Die gewünschte Beweglichkeit der Spannkörper gegenüber dem gemeinsamen Grundkörper kann insbesondere durch eine anforderungsgerechte geometrische Gestaltung erzielt werden, bspw. indem als Gelenk oder Scharnier dienende, bewegliche Bereiche etwas dünner gestaltet werden.

Die Spannkörper sind insbesondere Federzungen, insbesondere solche, die einseitig befestigt sind und daher ein freies, bewegliches Ende besitzen, das gegen das Endoskoprohr gedrängt werden kann. Die Verlagerung der Spannkörper zum Spannen entsteht durch einen Wirkeingriff in einer Auflauffläche einer axial gegenüber dem Grundkörper verlagerbaren Spannhülse. Eine solche Spannhülse, die auch als Schiebehülse bezeichnet werden kann, könnte einen konstanten inneren Durchmesser besitzen, während die einzelnen Spannkörper einen äußeren Durchmesser haben, der Längsrichtung zunimmt und dabei größer als der innere Durchmesser des Spannhülse wird. In Längsrichtung betrachtet kann der Querschnitt der Spannkörper keilförmig sein. Wird die Spannhülse mit konstantem Durchmesser über die keilförmigen Spannkörper geschoben, kommt die Spannhülse mit den Spannkörpern in Kontakt. Die Spannkörper nach innen gedrängt. Die Spannvorrichtung wird gespannt. Ein Endoskoprohr wird klemmend gehalten.

Umgekehrt ist es auch möglich, eine Spannhülse mit einer Auflauffläche zu verwenden, die sich trichterförmig, insbesondere konisch verjüngt, während der äußere Durchmesser der Spannkörper gleichbleibt. In diesem Fall wird die Spannhülse mit der sich verjüngenden, insbesondere konischen Auflauffläche nach dem Keilprinzip über die Spannkörper bewegt, um diese nach innen zu drängen bzw. um diese beim Zurückverlagern der Spannhülse wieder frei zu geben.

Die Spannhülse weist einen Gewindeabschnitt auf, welcher mit einem Gewindeabschnitt des Endoskopkopfes im Eingriff steht. Das Gewinde ist bevorzugt selbsthemmend. Die Spannhülse kann eine Überwurfmutter sein. Vorzugsweise wird jedoch das distale Ende des Endoskopkopfes von den Spannkörpern gebildet, während die Spannhülse zum Spannen vom proximalen zum distalen Ende verlagert wird und dabei die Spannkörper zusammendrückt. Der Innendurchmesser der Spannhülse oder Gewindehülse ist bevorzugt größer als der Außendurchmesser der radial nach innen verlagerten Spannkörper. Falls notwendig, kann die Spannhülse oder Gewindehülse in Längsrichtung geteilt sein, wobei die Teile nach der Montage auf dem Grundkörper miteinander verbunden werden.

Die Kraftübertragung von der Spannhülse auf die Spannkörper erfolgt nach dem Prinzip der schiefen Ebene. Dabei wird über eine vorwärts gerichtete Kraft eine Normalkraft auf eine Ebene ausgeübt. Die Kraftrichtung bzw. die Größe der Normalkraft hängt von dem Winkel der schiefen Ebene ab. Die Spannhülse selbst kann zu diesem Zweck eine geneigte, insbesondere konische Auflauffläche besitzen. Das ist allerdings nicht zwingend erforderlich. Die Spannkörper besitzen sie vorzugsweise Außenseiten, die bereits schräg zur Längsachse des Längskanals stehen. Es ist im Rahmen der Erfindung möglich, die Spannhülse und/oder die Spannkörper mit geeigneten Schrägflächen zu versehen, um eine an die jeweilige Spannsituation angepasste Kraftübertragung einzustellen.

Es gibt Anwendungsfälle, wie zum Beispiel bei der Bronchoskopie, bei denen es erforderlich ist, im Endoskoprohr (Bronchoskoprohr) zusätzliche Messkanäle vorzusehen. Ein oder mehrere Messkanäle müssen dementsprechend in den Endoskopkopf münden um bspw. den Druck am distalen Ende des Endoskoprohrs über ein Messmittel zu erfassen, das an den Endoskopkopf angeschlossen ist. In vorteilhafter Weiterbildung der Erfindung ist daher vorgesehen, dass die jeweilige Spannhülse wenigstens einen Kanal aufweist mit einem radial außen offenen Ende und einem radial innen offenen Ende. Das innere Ende des Kanals in der Spannhülse ist dazu vorgesehen, im gespannten Zustand der Spannvorrichtung fluidleitend mit einem Kanal verbunden zu sein, welcher in wenigstens einem Spannkörper angeordnet ist und nach radial innen zu einem aufzunehmenden Endoskoprohr offen ist.

Dadurch, dass diese beiden Kanäle in der Spannsituation so positioniert werden, dass sie fluidleitend miteinander verbunden sind, kann in dem Endoskopkopf ein im Wesentlichen radialer Durchgang von außen nach innen geschaffen werden, damit eine Verbindung zwischen dem Messkanal im Endoskoprohr und einem Messgerät am Endoskopkopf besteht.

Es ist aufgrund der beweglichen Spannhülse und dem beweglichen Spannkörper nicht trivial, die einzelnen Kanalöffnungen so zu platzieren, dass mit der notwendigen Sicherheit eine Fluidleitung möglich ist. Es müssen enge Toleranzen eingehalten werden. Gleichzeitig muss sichergestellt werden, dass die Spannhülse mit ihrem radial inneren, offenen Ende exakt über dem äußeren Ende des Kanals im Spannkörper angeordnet ist und selbstverständlich muss auch das Endoskoprohr in der richtigen Winkellage im Endoskopkopf angeordnet sein. Bevorzugt sind an dem Endoskopkopf entsprechende Markierungen oder auch mechanische Rastungen angeordnet, um sicherzustellen, dass in Abhängigkeit vom Durchmesser des Endoskoprohrs nur bestimmte Stellungen der Spannhülse eingestellt werden, nämlich ausschließlich solche, in denen eine fluidleitende Verbindung zwischen den Kanälen möglich ist.

Die Spannhülsen, die rotatorisch auf dem Grundkörper bewegt werden, weil sie über einen Gewindeabschnitt gelagert sind, haben Kanäle, welche in den Spannkörpern in Anpassung an die Steigung des Gewindeabschnitts in Axialrichtung zueinander versetzt angeordnet sind. Ein Beispiel: Ein Endoskopkopf dient zur Aufnahme von zwei unterschiedlich dicken Endoskoprohren. Es ist bekannt, welche Positionen den Spannhülsen einnehmen müssen, um das jeweilige Endoskoprohr zu klemmen. In der ersten Position für das dünnere Endoskoprohr muss die Spannhülse weiter in Längsrichtung verschoben werden, also weiter gedreht werden als für das dickere Endoskoprohr. Daher ist ein Kanal in einem ersten Spannkörper vorsehen, der näher zum distalen Ende hin angeordnet ist, und ein zweiter Kanal in einem anderen Spannkörper, wobei der zweite Kanal nicht nur in einem anderen Umfangsbereich, sondern auch in einem anderen Abstand zum distalen Ende angeordnet ist. Die Position der Kanäle in den Spannkörpern, wird durch die Gewindesteigung bestimmt und durch die Durchmesser der verwendeten Endoskoprohre.

Gemäß einer weiteren vorteilhaften Ausgestaltung der Erfindung besitzt die Spannhülse radial außen wenigstens einen Anschlussstutzen. Dieser Anschlussstutzen dient zum Anschluss eines Messmittels. Daher sollte der wenigstens eine Kanal, der die Spannhülse radial durchsetzt, in den Anschlussstutzen münden. Er mündet vorzugsweise in dessen äußerem Ende. Der Anschlussstutzen kann gleichzeitig als Anzeigemittel für den Bediener des Endoskops dienen, um die korrekte Position des Spannkörpers in Relation zum Durchmesser des Endoskoprohrs zu überprüfen. Gleichzeitig kann der Anschlussstutzen aufgrund seiner Länge so konfiguriert sein, dass er als Hebel konfiguriert ist und dadurch die Bedienbarkeit der Spannhülse vereinfacht. Er muss hierfür hinreichend groß dimensioniert sein, damit er auch beim Spannen der Spannhülse nicht beschädigt wird.

Erfindungsgemäß ist vorgesehen, dass die Spannvorrichtung mit dem Endoskoprohr verdrehsicher koppelbar ist. Die Verdrehsicherung kann kraftschlüssig und/oder formschlüssig erfolgen, indem mindestens ein Vaterstück am Endoskoprohr und/oder Endoskopkopf mit wenigstens einem Mutterstück am Endoskoprohr und/oder am Endoskopkopf in Eingriff bringbar ist. Für verschiedene Anwendungen ist es erforderlich, das Endoskoprohr während der Benutzung in die gewünschte Position drehen zu können und auch um über das Endoskoprohr Kraft auf das distale Ende des Endoskoprohrs übertragen zu können. Das ist am zuverlässigsten über formschlüssige Verbindungen möglich. Die formschlüssige Verbindung kann durch die kraftschlüssige Verbindung gesichert werden. Ein Formschluss kann durch axialen und/oder radialen Eingriff der miteinander in Eingriff stehenden Bauteile erfolgen.

Hierzu können am proximalen Ende des Endoskoprohrs bzw. im Endbereich des Endoskoprohrs entsprechende Aussparungen im Sinne eines Mutterstückes vorgesehen sein, die mit entsprechenden Vorsprüngen am Endoskopkopf korrespondieren (Vaterstück).

Gemäß einer weiteren Ausführungsform der Erfindung sind mehrere Endoskoprohre vorgesehen, die nacheinander mit dem Endoskoprohr koppelbar sind. Im praktischen Einsatz soll der Durchmesser der nacheinander verwendeten Endoskoprohre bevorzugt gesteigert werden. Dieser Fall wird nachfolgend beschrieben, wobei angemerkt wird, dass eine Verwendung des Endoskopkopfes von dicken Endoskoprohren hin zu dünneren Endoskoprohren möglich ist. In jedem Fall dient ein erstes Endoskoprohr als Führungsendoskoprohr. Zusätzlich zu dem Führungsendoskoprohr ist wenigstens ein weiteres Endoskoprohr vorgesehen. Die Endoskoprohre können aus einem formfesten Kunststoff bestehen. Jedes der Endoskoprohre besitzt einen Arbeitskanal und ist dazu ausgebildet, dass erste Endoskoprohr oder ein im Durchmesser passendes weiteres Endoskoprohr koaxial in sich aufzunehmen oder in einem solchen aufgenommen zu werden. Es handelt sich daher um einen Satz von Endoskoprohren, die im Durchmesser aufeinander abgestimmt sind. Die Endoskoprohre sind dafür vorgesehen, nacheinander vorzugsweise mit zunehmenden Durchmessern der Arbeitskanäle koaxial in einen zu endoskopierenden Hohlraum eingeführt zu werden. Eines der weiteren Endoskoprohre ist dabei über ein im Durchmesser kleineres Endoskoprohr steckbar, so dass das im Durchmesser jeweils kleinere Endoskoprohr jeweils das Führungsendoskoprohr für das weitere Endoskoprohr ist. Das jeweils als Führungsendoskoprohr dienende, innere der Endoskoprohre ist dazu ausgebildet, nach dem Aufstecken des weiteren Endoskoprohrs aus diesem herausgezogen zu werden, um den Arbeitskanal des weiteren Endoskoprohrs frei zu geben, wobei das jeweils äußere Endoskoprohr mit dem Endoskopkopf koppelbar ist. Das setzt eine komplette Durchgängigkeit des Arbeitskanals des größeren Endoskoprohres für das darin angeordnete kleinere Endoskoprohr voraus.

Eine solche Vorrichtung kann zwei bis sechs Endoskope umfassen, die aufeinander abgestimmt sind. Jedes nachfolgende, weitere Endoskop besitzt aufgrund des größeren Querschnitts bei gleichem Werkstoff und gleicher Wanddicke eine größere Biegesteifigkeit als das erste Endoskop. Die Endoskope bestehen vorzugsweise aus einem semi-rigiden bzw. formfesten Kunststoff. Es ist möglich, Endoskoprohre aus unterschiedlichen Werkstoffen zu verwenden. Die Endoskope aus Kunststoff können als Einmalendoskope zur Verfügung gestellt werden, um nach der Untersuchung bzw. Behandlung entsorgt zu werden.

Die Verwendung der erfindungsgemäßen Vorrichtung hat den Vorteil, dass zunächst mit Geräten gearbeitet werden kann, die einen kleinen Querschnitt haben, die flexibler sind, und dass der Querschnitt stufenweise gesteigert werden kann, ohne dass für jedes weitere Gerät ein eigener Endoskopkopf erforderlich ist. Eine Schnellkupplung durch die klemmend wirkenden Spannelemente ermöglicht einen raschen Austausch und minimiert die Behandlungszeit. Die Vorrichtung kann insbesondere auch in der Weise verwendet werden, dass bei Bedarf von einem dickeren Endoskoprohr auf ein dünneres Endoskoprohr gewechselt werden kann, z.B. wenn sich zeigt, dass mit dem dickeren Endoskoprohr eine Engstelle nicht passiert werden kann. Das dickere Endoskoprohr dient in diesem Fall als Führungsendoskop für das dünnere Endoskoprohr. Der Endoskopkopf kann auch bei dieser Vorgehensweise schnell und sicher jeweils von dem vorhergehend verwendeten Endoskoprohr gelöst werden und mit dem neuen Endoskoprohr verbunden werden. Das nicht beanspruchte Verfahren zeichnet sich durch folgende Schritte aus
a) Es wird ein erstes Endoskoprohr in einen zu endoskopierenden Hohlraum eingeschoben und mit dem Endoskopkopf verbunden;
b) Es wird ein zweites Endoskoprohr, dessen Durchmesser vom Durchmesser des ersten Endoskoprohrs abweicht, koaxial zum ersten Endoskoprohr in den Hohlraum eingebracht, nachdem der Endoskopkopf von dem ersten Endoskoprohr entfernt wurde;
c) Das erste Endoskoprohr wird entfernt und das im Hohlraum verbleibende Endoskoprohr wird mit dem Endoskopkopf verbunden. Bevorzugt wird bei diesem Verfahren ein im Durchmesser größeres Endoskoprohr über ein im Durchmesser kleineres Endoskoprohr geschoben.

Die Erfindung wird nachfolgend anhand eines in rein schematischen Zeichnungen dargestellten Ausführungsbeispieles (Figuren 1 bis 8) erläutert. Die Figuren 9 bis 11 zeigen eine nicht erfindungsgemäße Bauform. Die Figuren 9 bis 11 dienen der weiteren Illustration der Erfindung. Es zeigen:
- Figur 1: eine Seitenansicht eines Endoskopkopfes;
- Figur 2: den Endoskopkopf der Figur 1 in einer weiteren Seitenansicht;
- Figur 3: eine perspektivische Ansicht des Endoskopkopfes des Figur 1 ;
- Figur 4: den Endoskopkopf der Figuren 1 bis 3 in einer ersten Stirnansicht auf das proximale Ende;
- Figur 5: eine Stirnansicht auf das distale Ende des Endoskopkopfes der Figuren 1 bis 4;
- Figur 6: eine perspektivische Ansicht einer Spannhülse;
- Figur 7: die Spannhülse der Figur 6 in einer Seitenansicht;
- Figur 8: die Spannhülse der Figuren 6 und 7 in einer Stirnansicht;
- Figur 9: eine weitere Ausführungsform einer Spannhülse (nicht erfindungsgemäß) in einer Ansicht auf ihr distales Ende;
- Figur 10: eine Ansicht auf die Spannhülse der Figur 9 von der Seite und
- Figur 11: eine perspektivische Ansicht der Spannhülse der Figuren 9 und 10.

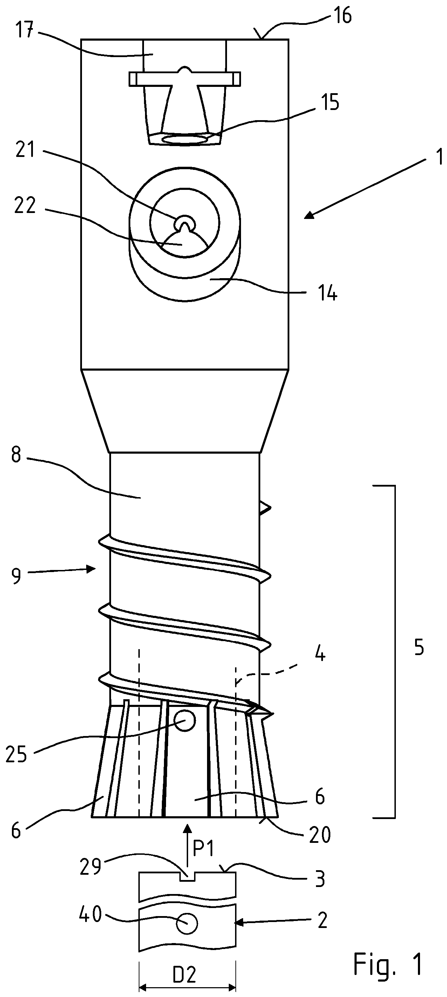

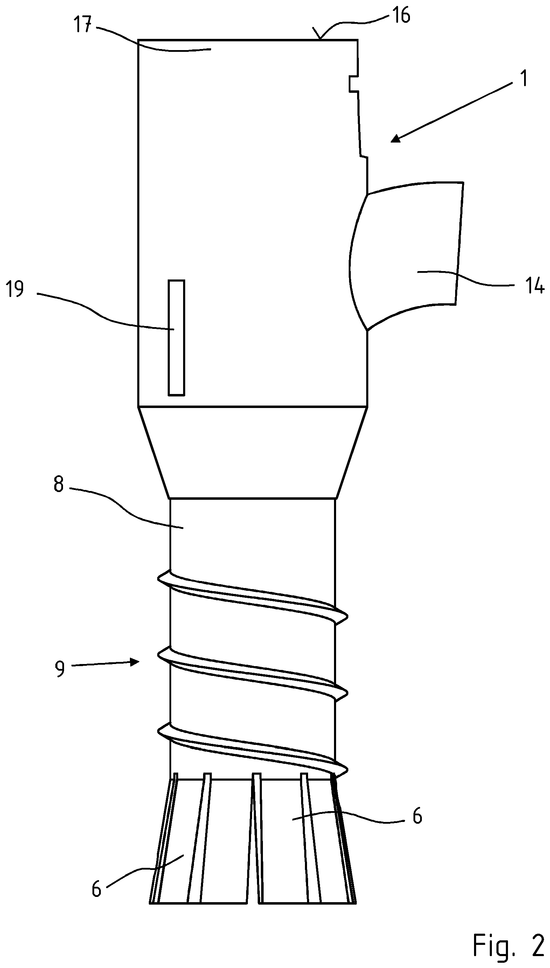

Die Figur 1 zeigt einen Endoskopkopf 1 als Bestandteil einer medizinischen Vorrichtung. Derselbe Endoskopkopf 1 ist in verschiedenen Ansichten auch in den Figuren 2 bis 5 dargestellt.

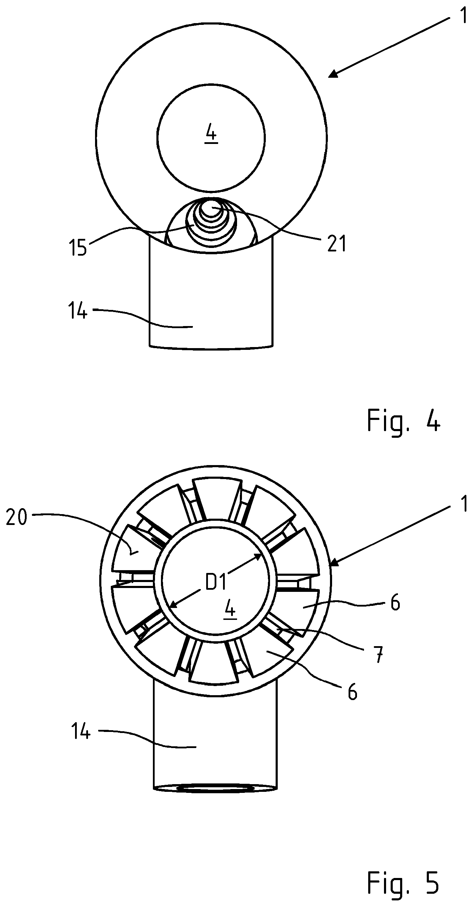

Der Endoskopkopf 1 ist dazu ausgebildet, mit einem Endoskoprohr 2 verbunden zu werden. Das Endoskoprohr 2 ist lediglich in Figur 1 rein schematisch und verkürzt dargestellt. Die Figur 1 zeigt das proximale Ende des Endoskoprohrs 2. Es wird in Richtung des Pfeils P1 in das distale Ende 20 des Endoskopkopfes 1 eingeführt. Hierzu befindet sich innerhalb des Endoskopkopfes 1 ein Längskanal 4 (Figuren 4 und 5). Der Längskanal 4 durchsetzt den gesamten Endoskopkopf 1. Er dient als Arbeitskanal. Der Längskanal 4 ist mir unterbrochener Linie in der Figur 1 angedeutet. In nicht näher dargestellter Weise kann der Längskanal 4 Abstufungen aufweisen, z.B. um eine Einstecktiefe eines aufzunehmenden Endoskoprohres 2 zu begrenzen.

Der Endoskopkopf 1 besitzt eine Spannvorrichtung 5, deren Funktionsabschnitt in der Figur 1 gekennzeichnet ist. Zu der Spannvorrichtung 5 gehören mehrere Komponenten. Insbesondere gehört zu der Spannvorrichtung 5 eine Anordnung von mehreren verlagerbaren Spannkörpern 6. Die Figur 5 zeigt die Spannkörper 6 in einer Stirnansicht. Sie sind gleichmäßig verteilt über dem Umfang des Längskanals 4 angeordnet. Bei diesem Ausführungsbeispiel handelt es sich um 10 Spannkörper. In der Praxis sind es mindestens drei über den Umfang verteilt, insbesondere gleichmäßig verteilt angeordnete Spannkörper, um eine Zentrierung eines Endoskoprohrs 2 zu ermöglichen. Die einzelnen Spannkörper 6 sind im Querschnitt im Wesentlichen keilförmig bzw. aufgrund der runden, radial äußeren Seite und radial inneren Seite kreisringsegmentförmig. Die einzelnen Spannkörper 6 sind identisch konfiguriert. Sie sind zu benachbarten Spannkörpern 6 im Abstand angeordnet. Zwischen jeweils zwei Spannkörpern 6 befindet sich daher ein Spalt 7. Er besitzt eine gleichbleibende Breite. Dieser Spalt 7 ist auch in den Darstellungen der Figuren 1 bis 3 zu erkennen. Er ermöglicht es, die zungenartigen Spannkörper 6 unabhängig voneinander zu bewegen und zum Spannen von außen nach innen zu verlagern.

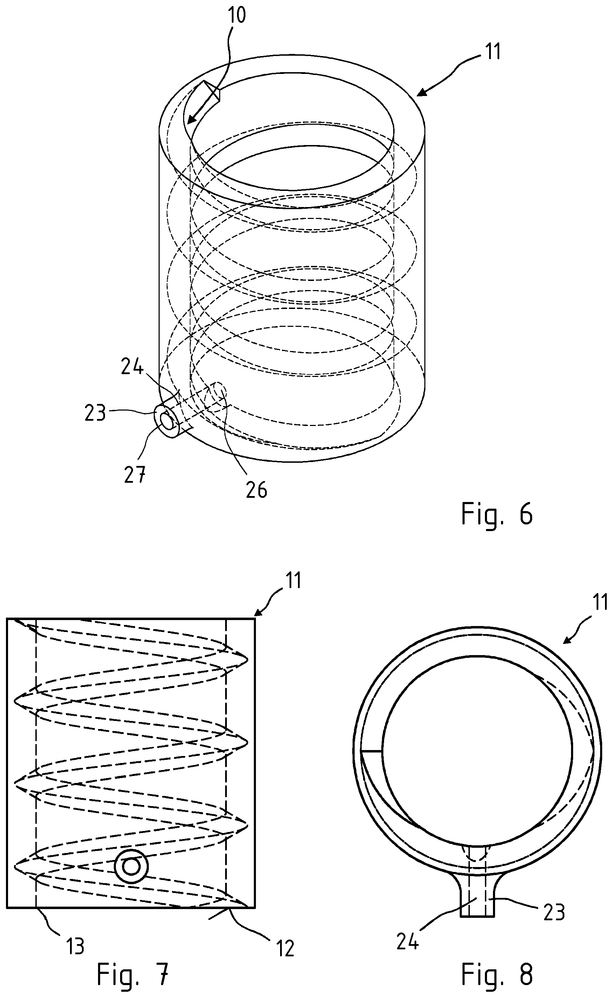

Die einzelnen Spannkörper 6 sind mit einem Grundkörper 8 verbunden. Der Grundkörper 8 ist zylindrisch. Bei diesem Ausführungsbeispiel ist der Grundkörper 8 materialeinheitlicher Bestandteil des Endoskopkopfes 1. An dem Grundkörper 8 ist ein Gewindeabschnitt 9 ausgebildet. Der Gewindeabschnitt 9 dient für den Eingriff mit einem Gewindeabschnitt 10 an einer Spannhülse 11, wie sie in den Figuren 6 bis 9 gezeigt ist. Die Spannhülse 11 besitzt dementsprechend ein Innengewinde, während der Gewindeabschnitt 9 auf den Grundkörper 8 ein Außengewinde ist. Durch Drehen der Spannhülse 10 relativ zum Endoskopkopf bzw. zum Gewindeabschnitt 9 auf dem Grundkörper 8 wird die Spannhülse 11 in Längsrichtung des Endoskopkopfes 1 verlagert und stößt hierbei auf die Spannkörper 6. Die Spannkörper 6 sind so konfiguriert, dass sie eine Öffnungsweite D1 freigeben, wie sie in der Figur 5 dargestellt ist. Die Spannkörper 6 befinden sich in den Figuren in einer Ausgangsposition. Sie sind entspannt. Wird nun die Spannhülse 11 durch Drehen auf dem Gewindeabschnitt 9 in Längsrichtung zum distalen Ende 20 des Endoskopkopfes 1 verlagert, stößt das distale Ende 12 der Spannhülse 11 auf die einzelnen Spannkörper 6. Die Spannkörper 6 besitzen in Längsrichtung einen keilförmigen Querschnitt. In der Ausgangsposition umgrenzen sie einen zylindrischen Innenraum, entsprechend dem kreisrunden Querschnitt des aufzunehmenden Endoskoprohrs 2. Radial außenseitig sind die mehreren Spannkörper 6 kegelstumpfartig ausgebildet in dem Sinne, dass jede einzelne Außenfläche der Spannkörper 6 ein Kegelstumpfmantelsegment beschreibt. Der äußere Durchmesser des von den Spannkörper 6 aufgespannten Kreises ist am distalen Ende des Endoskopkopfes 1 am größten und entspricht am proximalen Ende der Spannkörper 6 dem Außendurchmesser des zylindrischen Grundkörpers 8. Eine radial innere Kante des distalen Endes 12 der Spannhülse 11 dient als Auflauffläche 13 (Figur 7). Diese ringförmige Auflauffläche 13 ist die Fläche, an der sich die einzelnen Spannhülsen 6 abstützen und über welche die Spannhülsen 6 aktiv nach innen oder durch Rückfederung nach außen verlagert werden können, wenn die Spannhülse zurückgedreht wird. Dadurch ist es möglich, die variable Öffnungsweite D1 so einzustellen, dass Endoskoprohre 2 unterschiedlicher Durchmesser D2 in dem Längskanal 4 aufgenommen werden können und vor allen Dingen von der Spannvorrichtung 5 gehalten werden können.

Der Endoskopkopf 1 besitzt im Anschluss an den Grundkörper 8 bzw. im Anschluss an die Spannvorrichtung 5 zu seinem proximalen Ende 16 hin einen radial schräg abgehenden Anschlussstutzen 14 für ein Beatmungsgerät. Der Anschlussstutzen 14 ist längskanalisiert und mündet in den Längskanal 4. Der Anschlussstutzen 14 ist symbolisch dargestellt.

Darüber hinaus besitzt der Endoskopkopf 1 einen zweiten Anschluss 15 für eine Jet-Ventilation. Dieser Anschluss 15 liegt in Längsrichtung betrachtet fluchtend hinter dem Anschlussstutzen 14 für die Beatmung. Der Anschluss 15 mündet in einen Kanal, der im spitzen Winkel zur Längsachse des Endoskopkopfes 1 und damit zum Längskanal 4 verläuft. Daher mündet der Anschluss 15 auch stirnseitig parallel zum Längskanal 4 im proximalen Ende 16. Der Anschluss 15 selbst liegt in einer radialen, von außen eingebrachten Vertiefung 17. Diese radial und axial offene Vertiefung 17 am proximalen Ende 16 ist dem eigentlichen Anschluss 15 bzw. dem Kanal zur Jet-Ventilation vorgelagert. In dieser Vertiefung befindet sich eine quer zur Längsachse verlaufende Nut 18. In diese Nut 18 kann ein Sperrkörper (nicht dargestellt) eingesetzt werden. Der Sperrkörper stellt eine Barriere dar, die aktiv entfernt werden müsste um eine Jet-Ventilation durchzuführen. Das soll verhindern, dass bei einer Bronchoskopie eine Beatmung über den Anschlussstutzen 14 erfolgt und gleichzeitig eine Jet-Ventilation über den Anschluss 15. Der nicht näher dargestellte Sperrkörper kann bei Nichtgebrauch in einer Tasche 19, wie sie in Figur 2 zu erkennen ist, aufgenommen werden. Die Tasche 19 befindet sich auf der dem Anschlussstutzen 14 bzw. dem Anschluss 15 für die Jet-Ventilation abgewandten Seite, das heißt diametral zu den Anschlussstutzen 14.

Die Figuren 4 und 5 zeigen den im Wesentlichen zylindrischen Aufbau des Endoskopkopfes 1, einmal mit Blickrichtung auf sein proximales Ende 16 und einmal mit Blickrichtung auf sein distales Ende 20. Die Figur 4 zeigt zudem einen Kanal 21 zur Jet-Ventilation innerhalb des Anschlusses 15. Der Kanal 21 zur Jet-Ventilation mündet in den Beatmungskanal 22 im Anschlussstutzen 14, wie die Figur 1 zeigt. Der Kanal 21 mündet gewissermaßen im Übergangsbereich zum Längskanal 4 des Endoskopkopfes 1.

Der Spannköper 11, wie in den Figuren 6 bis 8 gezeigt ist, besitzt einen Anschlussstutzen 23. Der Anschlussstutzen 23 befindet sich benachbart zum distalen Ende 12 und steht radial ab. Innerhalb des Anschlussstutzens 23 befindet sich ein Kanal 24. Der Kanal 24 verläuft radial von innen nach außen. Je nach Stellung des Spannkörpers 11 auf dem Gewindeabschnitt 9 des Grundkörpers 8 befindet sich der Anschlussstutzen 23 und damit auch der darin angeordnete Kanal 24 in unterschiedlichen Positionen. Der Anschlussstutzen 23 dient zur Fluiddurchleitung, insbesondere zur Gasmessung in Verbindung mit einem Endoskoprohr 2, das in dem Endoskopkopf 1 aufgenommen ist. Hierzu befindet sich im Endoskoprohr 2 eine Messöffnung 40. Diese Messöffnung 40 wird beim Einsetzen des Endoskoprohrs 2 so positioniert, dass die Messöffnung 25 fluidleitend mit einem Kanal 25 verbunden ist, der sich in einem der Spannkörper 6 befindet. Der Kanal 25 in Spannkörper 6 muss wiederum mit dem Kanal 24 im Anschlussstutzen 23 der Spannhülse 11 fluidleitend verbunden sein. Hierzu wird die Spannhülse 11 so gedreht, dass das radial innere, offene Ende 26 des Kanals 24 in Überdeckung mit dem Kanal 25 im Spannkörper 6 gebracht wird. An das radial äußere, offene Ende 28 des Kanals 24 im Anschlussstutzen 23 kann dann eine Messeinrichtung angeschlossen werden, bspw. um den Luftdruck zu messen.

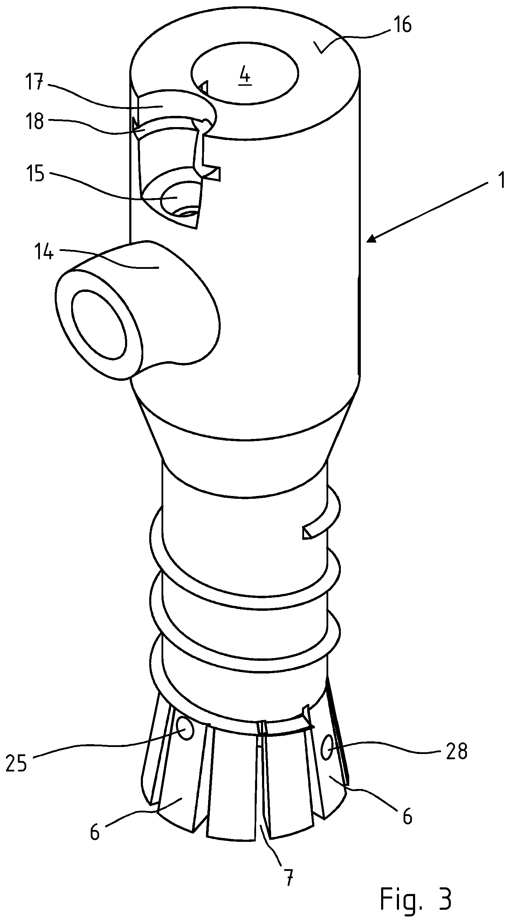

Aufgrund der Tatsache, dass bei unterschiedlichen Durchmessern der Endoskoprohre 2 auch die Spannhülse 11 in unterschiedlichen Positionen angeordnet ist, müssen auch entsprechende Kanäle 25, 28 in den Spannkörpern 6 vorgesehen sein. Die Figur 3 zeigt, dass mehrere Kanäle 25, 28 in unterschiedlichen axialen Längenabschnitten der Spannkörper 6 angeordnet sind. Dadurch kann sichergestellt werden, dass Kanal 24 in der Spannhülse 11 in fluidleitende Verbindung mit einem Kanal 25, 28 in den Spannkörpern 6 gebracht werden kann.

Um sicherzustellen, dass das Endoskoprohr 2 bezüglich der Messöffnung in der richtigen Position angeordnet ist, ist am proximalen Ende des Endoskoprohrs 2 ein fomschlüssiges Mittel zur Positionierung angeordnet. Es kann als Vaterstück oder Mutterstück ausgebildet sein. In diesem Fall ist es ein Mutterstück 29 in Form einer Vertiefung im proximalen Ende des Endoskoprohrs, wie es beispielhaft in Figur 1 gezeigt ist. Über dieses Mutterstück 29, das mit einem nicht näher dargestellten Vaterstück im inneren des Endoskopkopfes 1 korrespondiert, kann zudem eine Verdrehsicherung gewährleistet werden, um das Endoskoprohr 2 nicht nur kraftschlüssig durch Klemmung im Endoskopkopf 1 zu halten, sondern auch formschlüssig. Es wird die Einstecktiefe in den Endoskopkopf 1 definiert und damit die exakte Lage der Messöffnung 40 relativ zum Kanal 25 in dem Spannkörper 6. Zudem kann über den Endoskopkopf 1 ein Drehmoment auf das Endoskoprohr 2 ausgeübt werden.

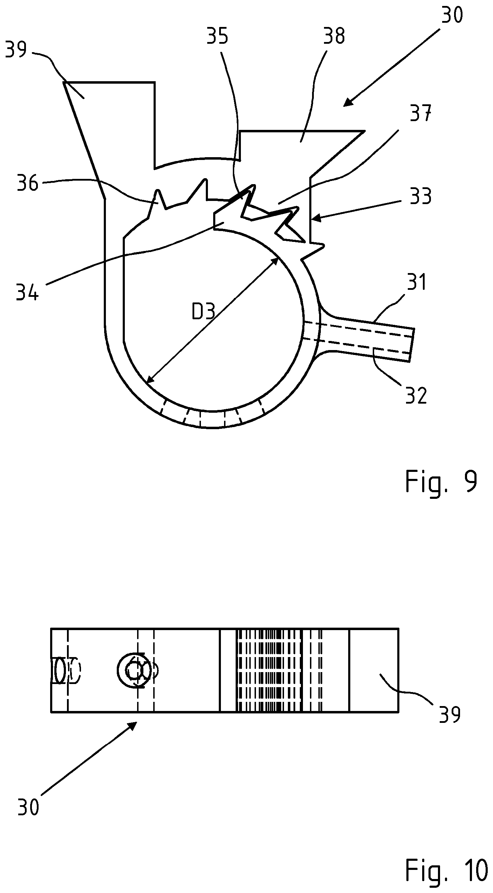

Die Figuren 9 bis 11 zeigen eine alternative Spannhülse 30, die nicht erfindungsgemäß ausgebildet ist. Diese Bauform der Spannhülse 30 kann ebenfalls mit einem Endoskopkopf 1, wie er in den Figuren 1 bis 5 gezeigt ist, verwendet werden. Die Spannhülse 30 besitzt wiederum einen Anschlussstutzen 31 mit einem Kanal 32 in Analogie zu dem Anschlussstutzen 23 mit dem Kanal 24 der Spannhülse 11 gemäß Figur 8. Der wesentliche Unterschied gemäß der zuerst erläuterten Spannhülse (Figuren 6 bis 8) ist, dass diese Spannhülse 30 keinen konstanten Innendurchmesser besitzt, sondern, dass der Durchmesser D3 veränderbar ist. Dadurch, dass die Spannhülse 30 nicht in Längsrichtung verlagert wird, verbleibt der Kanal 32 im Anschlussstutzen 23 an derselben Stelle. Es muss lediglich darauf geachtet werden, dass die Spannhülse 30 dieser Bauart im richtigen Längenabschnitt platziert ist. Hierzu können entsprechende Positionierungshilfen an den Spannkörpern 6 vorgesehen sein.

Die Spannkörper 6 werden bei Verringerung des Durchmessers D3 des Spannringes zusammengedrückt und bei Vergrößern des Durchmessers D3 entsprechend freigegeben. Die Spannhülse 30 besitzt eine Arretiervorrichtung 33, die es ermöglicht, die Spannhülse 30 auf vorgegebene, unterschiedliche Durchmesser einzustellen. Hierzu befinden sich an einem innenliegenden Endabschnitt 34 der Spannhülse 33 nach radial außen gerichtete Rastnasen 35. Bei diesem Beispiel handelt es sich um drei identische Rastnasen 35. Diese Rastnasen 35 sind dafür vorgesehen, in Rastausnehmungen 36 eines äußeren Endabschnitts 37 der Spannhülse 30 zu fassen. Es sind zwei Rastnasen 35 mit zwei Rastausnehmung 36 im Eingriff. Dadurch ergeben sich bei der hier gewählten Geometrie drei unterschiedlich einstellbare Durchmesserbereiche. Wenn es ausreicht, dass nur eine Rastnase 35 mit einer Rastausnehmung 36 im Eingriff ist, ergeben sich sogar vier einstellbare Durchmesser, die durch Verstellen der Rastpositionen wählbar sind.

Das Verstellen der Durchmesser hin zu kleineren Durchmesserbereichen ist dadurch vereinfacht, dass die Rastnasen 35 und auch die Rastausnahmen 36 einseitig abgeschrägt sind, entsprechend eines Sägezahn-Designs. Die steileren Flanken der Rastnasen 35, die insbesondere radial zur Längsachse der Spannhülse 30 verlaufen, verhindern ein unbeabsichtigtes Öffnen.

Am radial außen liegenden äußeren Endabschnitt 37 befindet sich ein erster Handhabungsabschnitt 38, der das Greifen des äußeren Endabschnitts 37 der Spannhülse 30 vereinfacht, um die Spannhülse 30 zu lösen. Ein weiterer radial nach außen vorstehender Handhabungsabschnitt 39 befindet sich im Abstand vom ersten Handhabungsabschnitt 38 und dient zur Handhabung während des Spannens der Spannhülse 30.

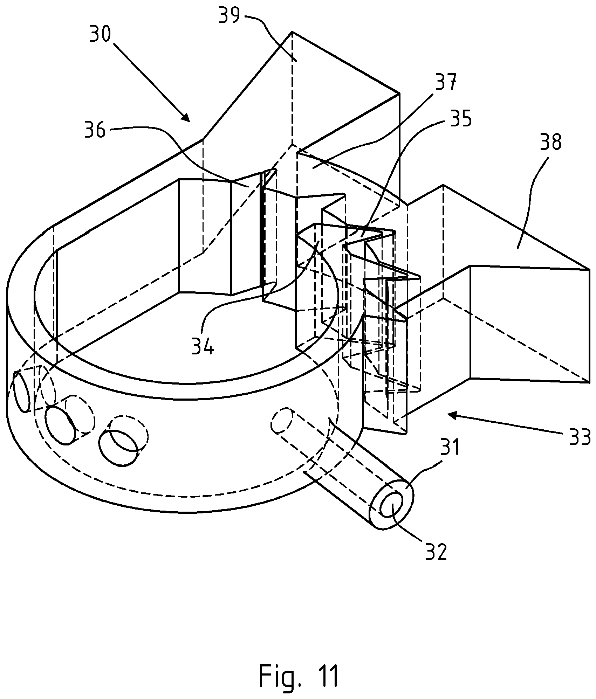

Weitere Einzelheiten der Spannhülse 30 sind anhand der Figuren 10 und 11 zu erkennen.

### Bezugszeichen:

- 1 -: Endoskopkopf
- 2 -: Endoskoprohr
- 3 -: proximales Ende von 2
- 4 -: Längskanal von 1
- 5 -: Spannvorrichtung
- 6 -: Spannkörper
- 7 -: Kanal zwischen 6
- 8 -: Grundkörper
- 9 -: Gewindeabschnitt an 8
- 10 -: Gewindeabschnitt an 11
- 11 -: Spannhülse
- 12 -: distales Ende von 11
- 13 -: Auflauffläche an 11
- 14 -: Anschlussstutzen
- 15 -: Anschluss für Jet-Ventilation
- 16 -: proximales Ende von 1
- 17 -: Vertiefung in 1
- 18 -: Nut in 17
- 19 -: Tasche in 1
- 20 -: distales Ende von 1
- 21 -: Kanal für Jet-Ventilation
- 22 -: Kanal zur Ventilation
- 23 -: Anschlussstutzen
- 24 -: Kanal in 23
- 25 -: Kanal in 6
- 26 -: inneres Ende von 24
- 27 -: äußeres Ende von 24
- 28 -: Kanal in 6
- 29 -: Mutterstück in 2
- 30 -: Spannhülse
- 31 -: Anschlussstutzen
- 32 -: Kanal in 31
- 33 -: Arretiermittel
- 34 -: innerer Endabschnitt von 30
- 35 -: Rastnase an 34
- 36 -: Rastausnehmung an 37
- 37 -: äußerer Endabschnitt von 30
- 38 -: erstes Handhabungsmittel an 30
- 39 -: zweites Handhabungsmittel
- 40 -: Messöffnung in 2

- D1 -: Öffnungsweite von 4
- D2 -: Durchmesser von 2
- D3 -: Durchmesser von 30
- P1 -: Pfeil

## Patentansprüche

1. Medizinische Vorrichtung mit einem Endoskopkopf (1), der dazu ausgebildet ist, mit Endoskoprohren (2) lösbar verbunden zu werden, wobei der Endoskopkopf (1) einen Längskanal (4) zur Aufnahme eines Endoskoprohrs (2) besitzt, wobei der Längskanal (4) eine variable Öffnungsweite (D1) zur Aufnahme von Endoskoprohren (2) unterschiedlicher Durchmesser (D2) besitzt und wobei der Endoskopkopf (1) eine Spannvorrichtung (5) besitzt, um ein in den Längskanal (4) eingesetztes Endoskoprohr (2) klemmend zu halten, wobei die Spannvorrichtung (5) mehrere verlagerbare Spannkörper (6) aufweist, die über den Umfang des Längskanals (4) verteilt angeordnet sind und von radial außen gegen ein Endoskoprohr (2) drängbar sind, wobei die Spannkörper (6) beweglich mit einem gemeinsamen Grundkörper (8) verbunden sind, **dadurch gekennzeichnet, dass** die Spannkörper (6) zum Spannen im Wirkeingriff mit einer Auflauffläche (13) einer axial gegenüber dem Grundkörper (8) verlagerbaren Spannhülse (11, 30) stehen, wobei die Spannhülse (11) einen Gewindeabschnitt (10) aufweist und mit einem Gewindeabschnitt (9) des Endoskopkopfes (1) in Eingriff steht.

2. Vorrichtung nach Anspruch 1 oder 2, **dadurch gekennzeichnet, dass** die Spannkörper (6) zum Spannen im Wirkeingriff mit einer Spannhülse (30) stehen, die einen in einem Kontaktbereich mit den Spannkörpern (6) veränderbaren inneren Durchmesser (D3) besitzt.

3. Vorrichtung nach Anspruch 1 bis 2, **dadurch gekennzeichnet, dass** die jeweilige Spannhülse (11, 30) wenigstens einen Kanal (24, 32) aufweist, mit einem radial außen offenen Ende (27 ) und mit einem radial innen offenen Ende (26), wobei das innere Ende (26) dazu vorgesehen ist, im gespannten Zustand der Spannvorrichtung (11, 30) fluidleitend mit einem Kanal (25, 28) verbunden zu sein, welcher in wenigstens einem Spannkörper (6) angeordnet ist und nach radial innen zu einem aufzunehmenden Endoskoprohr (2) offen ist.

4. Vorrichtung nach Anspruch 3, **dadurch gekennzeichnet, dass** die Kanäle (25, 28) in den Spannkörpern (6) in Anpassung an eine Steigung des Gewindeabschnittes (9) in Axialrichtung des Längskanals (4) zueinander versetzt angeordnet sind.

5. Vorrichtung nach Anspruch 4, **dadurch gekennzeichnet, dass** die Spannhülse (11, 30) radial außen wenigstens einen Anschlussstutzen (23, 31) aufweist, wobei der wenigstens ein Kanal (24, 32), der in der Spannhülse (11, 30) verläuft, in dem Anschlussstutzen (23, 31) mündet.

6. Vorrichtung nach einem der Ansprüche 1 bis 5, **dadurch gekennzeichnet, dass** die Spannkörper (6) fest mit dem Endoskopkopf (1) verbunden sind.

7. Vorrichtung nach einem der Ansprüche 1 bis 6, **dadurch gekennzeichnet, dass** der Endoskopkopf (1) mit dem Endoskoprohr (2) verdrehsicher koppelbar ist, wobei die Verdrehsicherung kraftschlüssig und/oder formschlüssig ausgebildet ist, indem mindestens ein Vaterstück am Endoskoprohr (2) und/oder am Endoskopkopf (1) mit wenigstens einem Mutterstück (29) am Endoskoprohr (2) und/oder am Endoskopkopf (1) in Eingriff bringbar ist.

8. Vorrichtung nach einem der Ansprüche 1 bis 7 aufweisend
a) ein erstes Endoskoprohr als Führungsendoskoprohr und
b) wenigstens ein weiteres Endoskoprohr, wobei das wenigstens eine weitere Endoskoprohr einen Arbeitskanal aufweist, und dazu ausgebildet ist, das erste Endoskoprohr oder ein im Durchmesser passendes weiteres Endoskoprohr koaxial in sich aufzunehmen oder in einem solchen aufgenommen zu werden,
c) wobei die Endoskoprohre dafür vorgesehen sind, nacheinander koaxial in einen zu endoskopierenden Hohlraum eingeführt zu werden, wobei eines der weiteren Endoskoprohre über ein im Durchmesser kleineres Endoskoprohr steckbar ist oder in ein im Durchmesser größeres Endoskoprohr steckbar ist, wobei ein Endoskoprohr eine Führung für das andere Endoskoprohr ist, und wobei
d) wobei das jeweils als Führungsendoskoprohr dienende Endoskoprohr dazu ausgebildet ist, nach dem koaxialen Einbringen des weiteren Endoskoprohrs zurückgezogen zu werden, wobei das in den Hohlraum eingeführte, verbleibende Endoskoprohr mit dem Endoskopkopf (1) koppelbar ist.

## Claims

1. A medical device with an endoscope head (1), that is configured to be detachably connected to endoscope tubes (2), wherein the endoscope head (1) has a longitudinal channel (4) for receiving an endoscope tube (2), wherein the longitudinal channel (4) has a variable opening width (D1) for receiving endoscope tubes (2) of different diameters (D2) and wherein the endoscope head (1) has a clamping device (5) for clamping an endoscope tube (2) inserted into the longitudinal channel (4), wherein the clamping device (5) has a plurality of displaceable clamping bodies (6) which are arranged distributed over the circumference of the longitudinal channel (4) and can be urged from the radial outside against an endoscope tube (2), wherein the clamping bodies (6) are movably connected to a common base body (8), **characterized in that** the clamping bodies (6) are in operative engagement with a run-up surface (13) of a clamping sleeve (11, 30) which is axially displaceable relative to the base body (8), wherein the clamping sleeve (11) has a threaded portion (10) and engages with a threaded portion (9) of the endoscope head (1).

2. The device according to claim 1 or 2, **characterized in that** the clamping bodies (6) are in operative engagement with a clamping sleeve (30) for clamping, which has an inner diameter (D3) that can be changed in a contact area with the clamping bodies (6).

3. The device according to claim 1 to 2, **characterized in that** the respective clamping sleeve (11, 30) has at least one channel (24, 32) with a radially outwardly open end (27) and with a radially inwardly open end (26), wherein the inner end (26) is provided, in the tensioned state of the tensioning device (11, 30), to be fluidly connected to a channel (25, 28), which is arranged in at least one tensioning body (6) and is open radially inwardly to an endoscope tube (2) to be received.

4. The device according to claim 3, **characterized in that** the channels (25, 28) in the clamping bodies (6) are arranged offset from one another in the axial direction of the longitudinal channel (4) in adaptation to a pitch of the threaded portion (9).

5. The device according to claim 4, **characterized in that** the clamping sleeve (11, 30) has at least one connecting piece (23, 31) radially outwardly, wherein the at least one channel (24, 32) running in the clamping sleeve (11, 30) opens into the connecting piece (23, 31).

6. The device according to any one of claims 1 to 5, **characterized in that** the clamping bodies (6) are firmly connected to the endoscope head (1).

7. The device according to any one of claims 1 to 6, **characterized in that** the endoscope head (1) can be coupled to the endoscope tube (2) in a rotationally secure manner, wherein the rotational security is configured to be non-positive and/or positive, **in that** at least one male piece on the endoscope tube (2) and/or on the endoscope head (1) can be brought into engagement with at least one female piece (29) on the endoscope tube (2) and/or on the endoscope head (1).

8. The device according to any one of claims 1 to 7, having
a) a first endoscope tube as a guide endoscope tube, and
b) at least one further endoscope tube, wherein the at least one further endoscope tube has a working channel and is configured to coaxially accommodate the first endoscope tube or a further endoscope tube of matching diameter or to be accommodated in such a tube;
c) wherein the endoscope tubes are intended to be inserted coaxially one after the other into a cavity to be endoscoped, wherein one of the further endoscope tubes can be plugged over an endoscope tube of smaller diameter or can be plugged into an endoscope tube of larger diameter, wherein one endoscope tube is a guide for the other endoscope tube, and wherein
d) wherein the endoscope tube serving as the guide endoscope tube is configured to be withdrawn after the coaxial introduction of the further endoscope tube, wherein the remaining endoscope tube, introduced into the cavity, can be coupled to the endoscope head (1).

## Revendications

1. Dispositif médical avec une tête (1) d'endoscope qui est réalisée pour être reliée de manière amovible à des tubes (2) d'endoscope, dans lequel la tête (1) d'endoscope possède un canal longitudinal (4) pour recevoir un tube (2) d'endoscope, dans lequel le canal longitudinal (4) possède une largeur d'ouverture variable (D1) pour recevoir des tubes (2) d'endoscope de diamètres (D2) différents et dans lequel la tête (1) d'endoscope possède un dispositif de mise sous tension (5) pour maintenir par serrage un tube (2) d'endoscope inséré dans le canal longitudinal (4), dans lequel le dispositif de mise sous tension (5) présente plusieurs corps de mise sous tension (6) déplaçables qui sont disposés de manière répartie sur la périphérie du canal longitudinal (4) et peuvent être poussés depuis l'extérieur radialement contre un tube (2) d'endoscope, dans lequel les corps de mis sous tension (6) sont reliés de manière mobile à un corps de base commun (8), **caractérisé en ce que** les corps de mise sous tension (6) se trouvent pour la mise sous tension en prise active avec une surface d'appui (13) d'une douille de mise sous tension (11, 30) déplaçable axialement par rapport au corps de base (8), dans lequel la douille de mise sous tension (11) présente une partie filetée (10) et est en prise avec une partie filetée (9) de la tête (1) d'endoscope.

2. Dispositif selon la revendication 1 ou la revendication 2, **caractérisé en ce que** les corps de mise sous tension (6) se trouvent pour la mise sous tension en prise active avec une douille de mise sous tension (30) qui possède un diamètre intérieur (D3) modifiable dans une zone de contact avec les corps de mise sous tension (6).

3. Dispositif selon la revendication 1 à 2, **caractérisé en ce que** la douille de mise sous tension (11, 30) respective présente au moins un canal (24, 32), avec une extrémité ouverte à l'extérieur radialement (27) et avec une extrémité ouverte à l'intérieur radialement (26), dans lequel l'extrémité intérieure (26) est prévue pour être reliée, à l'état sous tension du dispositif de mise sous tension (11, 30), de manière conductrice de fluide à un canal (25, 28) qui est disposé dans au moins un corps de mise sous tension (6) et est ouvert vers l'intérieur radialement vers un tube (2) d'endoscope à recevoir.

4. Dispositif selon la revendication 3, **caractérisé en ce que** les canaux (25, 28) sont disposés de manière décalée l'un par rapport à l'autre dans les corps de mise sous tension (6) en adaptation à une pente de la partie filetée (9) dans le sens axial du canal longitudinal (4).

5. Dispositif selon la revendication 4, **caractérisé en ce que** la douille de mise sous tension (11, 30) présente à l'extérieur radialement au moins une tubulure de raccordement (23, 31), dans lequel l'au moins un canal (24, 32), qui s'étend dans la douille de mise sous tension (11, 30), débouche dans la tubulure de raccordement (23, 31).

6. Dispositif selon l'une quelconque des revendications 1 à 5, **caractérisé en ce que** les corps de mise sous tension (6) sont reliés de manière solidaire à la tête (1) d'endoscope.

7. Dispositif selon l'une quelconque des revendications 1 à 6, **caractérisé en ce que** la tête (1) d'endoscope peut être couplée au tube (2) d'endoscope de manière résistante à la rotation, dans lequel le dispositif anti-rotation est réalisé à force et/ou par complémentarité de forme, **en ce qu'**au moins une pièce mâle sur le tube (2) d'endoscope et/ou sur la tête (1) d'endoscope peut être amenée en prise avec au moins une pièce écrou (29) sur le tube (2) d'endoscope et/ou sur la tête (1) d'endoscope.

8. Dispositif selon l'une quelconque des revendications 1 à 7, présentant
a) un premier tube d'endoscope servant de tube de guidage d'endoscope et
b) au moins un autre tube d'endoscope, dans lequel l'au moins un autre tube d'endoscope présente un canal de travail, et est réalisé pour recevoir de manière coaxiale dans luimême le premier tube d'endoscope ou un autre tube d'endoscope correspondant en diamètre ou être reçu dans un tube d'endoscope de ce type ;
c) dans lequel les tubes d'endoscope sont prévus pour être introduits successivement de manière coaxiale dans une cavité à soumettre à une endoscopie, dans lequel un des autres tubes d'endoscope est enfichable sur un tube d'endoscope de diamètre plus petit ou est enfichable dans un tube d'endoscope de diamètre plus grand, dans lequel un tube d'endoscope est un guide pour l'autre tube d'endoscope, et dans lequel
d) dans lequel le tube d'endoscope servant respectivement de tube de guidage d'endoscope est réalisé pour être rétracté après l'insertion coaxiale de l'autre tube d'endoscope, dans lequel le tube d'endoscope restant introduit dans la cavité peut être couplé à la tête (1) d'endoscope.
